(19) Europäisches Patentamt
European Patent Office
Office européen des brevets

(11) Veröffentlichungsnummer: **0 073 506**
**B1**

(12) **EUROPÄISCHE PATENTSCHRIFT**

(45) Veröffentlichungstag der Patentschrift: **19.02.86**

(21) Anmeldenummer: **82107929.0**

(22) Anmeldetag: **28.08.82**

(51) Int. Cl.⁴: **C 07 D 487/04,** C 07 D 513/04, C 07 D 498/04, A 61 K 31/55 // (C07D487/04, 235:00, 223:00), (C07D513/04, 281:00, 235:00), (C07D498/04, 267:00, 235:00)

# (54) Heterocyclische Verbindungen, ihre Herstellung und Verwendung.

(30) Priorität: **02.09.81 DE 3134672**

(43) Veröffentlichungstag der Anmeldung: **09.03.83 Patentblatt 83/10**

(45) Bekanntmachung des Hinweises auf die Patenterteilung: **19.02.86 Patentblatt 86/8**

(84) Benannte Vertragsstaaten: **AT BE CH DE FR IT LI LU NL SE**

(56) Entgegenhaltungen:
**EP - A - 0 035 749**
**DE - A - 2 248 477**
**DE - A - 2 311 019**
**DE - A - 2 548 045**

**A-BURGER, MEDICINAL CHEMISTRY, FOURTH EDITION, 1979, Part III, 509, 517-519**

(73) Patentinhaber: **BOEHRINGER INGELHEIM KG, D-6507 Ingelheim am Rhein (DE)**

(72) Erfinder: **Walther, Gerhard, Dr., Pfarrer-Heberer-Strasse 37, D-6530 Bingen/Rhein (DE)**
Erfinder: **Schneider, Claus, Dr., Albrecht-Dürer-Strasse 19, D-6507 Ingelheim/Rhein (DE)**
Erfinder: **Weber, Karl-Heinz, Dr., Kaiser-Karl-Strasse 11, D-6535 Gau-Algesheim (DE)**
Erfinder: **Fügner, Armin, Dr., Im Hippel 31, D-6535 Gau-Algesheim (DE)**

**EP 0 073 506 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue heterocyclische Verbindungen, ihre Herstellung nach an sich bekannten Verfahren, ihre Verwendung als Arzneistoffe und die Herstellung entsprechender Arzneimittel.

Die neuen Verbindungen entsprechen der allgemeinen Formel

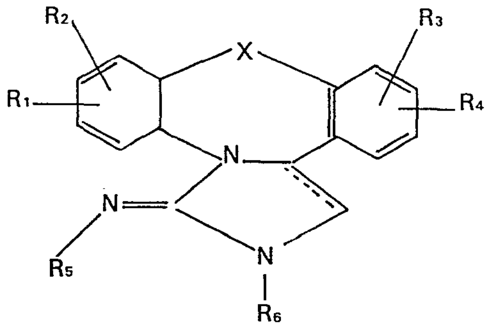

(I),

in der

$R_1$, $R_2$, $R_3$ und $R_4$ Wasserstoff, Halogen, Alkyl- oder Alkoxy mit jeweils 1 bis 6 C-Atomen,

$R_5$ Wasserstoff, Alkyl mit 1 bis 6 C-Atomen, oder Allyl,

$R_6$ Alkyl mit 1 bis 6 C-Atomen, Allyl, einen Rest der Formel

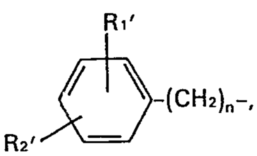

wobei $R_1'$ und $R_2'$ die gleichen Bedeutungen wie $R_1$ und $R_2$ annehmen können, aber nicht jeweils mit diesen identisch sein müssen, und n eine der ganzen Zahlen 0 bis 4 bedeutet,

X für O, S oder $CH_2$ steht und die gestrichelte Linie das Vorliegen entweder einer Einfach- oder einer Doppelbindung bedeuten kann.

Verbindungen, welche ein asymmetrisches C-Atom aufweisen, können als Racemate oder als reine Enantiomere bzw. als Gemische mit verschiedenen Anteilen der Enantiomeren auftreten. Alle Verbindungen können in Form der freien Basen oder der Säureadditionssalze vorliegen.

Die Reste $R_1$ bis $R_4$ bzw. $R_5$ und $R_6$ sowie $R_1'$ und $R_2'$ können untereinander gleich oder verschieden sein. Die Alkyl- bzw. Alkoxygruppen können geradkettig oder verzweigt sein. Dementsprechend haben die Alkylreste folgende Formeln:

$-CH_3, -C_2H_5, -CH_2-CH_2-CH_3, -CH(CH_3)_2,$
$-(CH_2)_3-CH_3, -CH_2-CH(CHH_3)_2,$
$-CH(CH_3)-C_2H_5, -C(CH_3)_3, -(CH_2)_4-CH_3,$
$-CH_2-CH_2-CH(CH_3)_2, -CH_2-CH(CH_3)-C_2H_5,$
$-CH(CH_3)-CH_2-CH_3, -CH_2-C(CH_3)_3,$
$-C(CH_3)_2-C_2H_5, -CH(CH_3)-CH(CH_3)_2,$
$-(CH_2)_5-CH_3, -(CH_2)_3-CH(CH_3)_2,$
$-CH_2-CH_2-CH(CH_3)-C_2H_5,$
$-CH_2-CH(CH_3)-CH_2-CH_2-CH_3, -CH(CH_3)-(CH_2)_3-CH_3, -C(CH_3)_2-CH_2-CH_2-CH_3,$
$-CH_2-C(CH_3)_2-C_2H_5, -CH_2-CH_2-C(CH_3)_3,$
$-CH(CH_3)-CH(CH_3)-C_2H_5, -CH(CH_3)-CH_2-CH(CH_3)_2, -C(CH_3)_2-C(CH_3)_2,$
$-CH(CH_3)-C(CH_3)_3, -CH_2-CH(C_2H_5)_2,$
$-CH(C_2H_5)-(CH_2)_2-CH_3.$

Die Alkoxyreste können dieselben Kohlenstoffskelette enthalten.

Die Bezeichnung der Positionen ist in der nachstehenden Skelett-Formel angegeben.

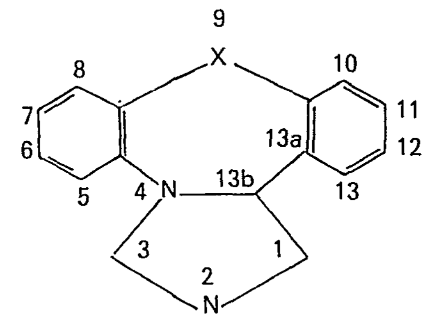

Zur Herstellung der Verbindungen der Formel I können folgende Verfahren benutzt werden:

1. Umsetzung eines Diamins der Formel II

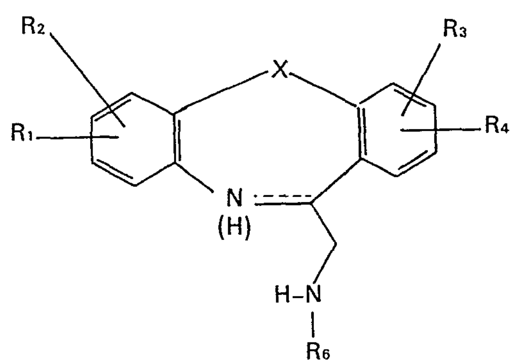

(II)

in welcher die gestrichelte Linie, die Reste $R_1$ bis $R_4$ und $R_6$ sowie X die obige Bedeutung haben, mit einem Halogencyan wie Bromcyan.

Man erhält Verbindungen der Formel I in denen $R_5$ Wasserstoff bedeutet.

Die Umsetzung erfolgt bevorzugt bei Raumtemperatur in einem Äthanol/Tetrahydrofuran-Gemisch, kann aber auch in anderen Lösungsmitteln wie Alkoholen, Chloroform oder Kohlenwasserstoffen wie Toluol, Xylol etc. gegebenenfalls auch unter Zusatz einer Hilfsbase (wie z.B. Kaliumcarbonat) durchgeführt werden. Die Reaktionstemperaturen sind weitgehend variabel und können bis zur Siedetemperatur des Reaktionsgemisches gehen.

Bei der Umsetzung einer Verbindung der Formel II mit z.B. Bromcyan bildet sich intermediär

eine Verbindung der Formel

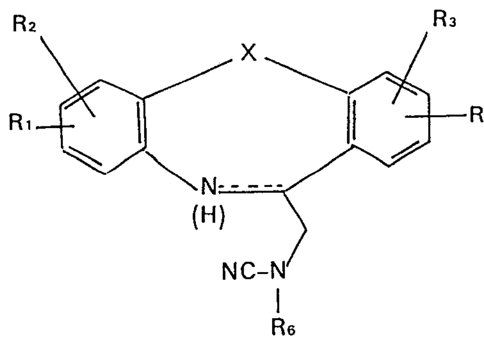

(IIa)

in der die gestrichelte Linie, die Reste $R_1$ bis $R_4$ und $R_6$ sowie X die obengenannte Bedeutung haben, die somit den eigentlichen Ausgangsstoff der Ringschluss-Reaktion bildet. Es ist jedoch nicht erforderlich, diese Verbindungen zu isolieren, da sie glatt zu dem gewünschten Endprodukt weiterreagiert.

Die Diamine der Formel II sind teilweise bekannt [s. J. Med. Chem. 13, 35–39 (1970); oder GB-PS 1 229 252] oder können analog hergestellt werden.

Aus den Endprodukten der Formel I mit $R_5 = H$, welche nach den vorstehend beschriebenen Verfahren erhältlich sind, lassen sich durch anschliessende Alkylierung bzw. Alkenylierung mit geeigneten Alkyl- bzw. Alkenylhalogeniden bzw. Sulfonsäureestern der Formel V

$$R_5\text{-}X \qquad (V)$$

in der $R_5$ die obengenannte Bedeutung (mit Ausnahme von Wasserstoff) hat, und X ein Halogenatom oder einen Sulfonyloxyrest, wie den Tosyloxy- oder Mesyloxyrest, bedeutet, in einem geeigneten, vorzugsweise polaren Lösungsmittel wie Dimethylformamid und in Gegenwart einer Base, wie Natriumhydrid, herstellen.

2. Ein weiteres Verfahren ist der Ringschluss einer Verbindung der Formel

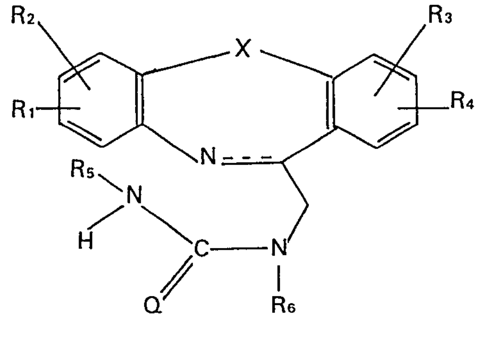

(VI)

in der $R_1$ bis $R_6$, die gestrichelte Linie und X die obige Bedeutung haben (wobei aber $R_5$ von Wasserstoff verschieden ist) und Q für Sauerstoff oder Schwefel steht.

Der Ringschluss zu den erfindungsgemässen Verbindungen der Formel I erfolgt durch Einwirkung von Säurehalogeniden, wie $POCl_3$, gegebenenfalls in gebräuchlichen Lösungsmitteln, wie Toluol. Eine Variante dieses Verfahrens besteht in der S-Alkylierung der Thioharnstoffderivate der Formel VI (d.h. Q gleich Schwefel) zu den entsprechenden Alkylmercapto-Verbindungen der Formel

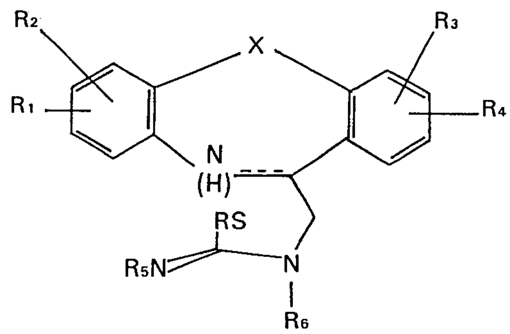

(VII)

(gestrichelte Linie, $R_1$ bis $R_6$ und X wie oben angegeben, R gegebenenfalls substituierter Alkylrest mit 1 bis 4 C-Atomen), die in an sich bekannter Weise durch Erwärmen in gebräuchlichen Lösungsmitteln, z.B. Alkohol, Toluol, oder in der Schmelze zu Verbindungen der Formel I ringgeschlossen werden.

Die Ausgangsverbindungen der Formel VI werden nach üblichen Verfahren erhalten, z.B. durch Umsetzung von Diaminen der Formel II mit Alkylisocyanaten bzw. Alkylisothiocyanaten mit Alkylresten von 1 bis 4 C-Atomen.

Die nach den verschiedenen Verfahren erhaltenen Reaktionsprodukte werden mit Hilfe bekannter Labormethoden isoliert. Gegebenenfalls können die so erhaltenen Rohprodukte noch unter Verwendung besonderer Methoden, z.B. durch Säulenchromatographie, gereinigt werden, ehe man sie in Form der Basen oder geeigneter Salze kristallisiert.

Gewünschtenfalls werden erfindungsgemäss erhaltene Racemate nach üblichen Methoden in die Enantiomeren aufgetrennt.

Aus primär erhaltenen Salzen werden gewünschtenfalls freie Basen, aus primär erhaltenen freien Basen Säureadditionssalze nach üblichen Methoden gewonnen.

Zur Herstellung von Säureadditionssalzen werden insbesondere solche Säuren verwendet, die zur Bildung von therapeutisch verwendbaren Salzen geeignet sind.

Als solche Säuren seien beispielsweise genannt:

Halogenwasserstoffsäuren; aliphatische, alicyclische, aromatische oder heterocyclische Carbonsäuren oder Sulfonsäuren, wie Essigsäure, Weinsäure, Malonsäure, Zitronensäure, Fumarsäure, Salicylsäure, Urethansulfonsäure, Toluolsulfonsäure; ferner Schwefelsäure, Phosphorsäure.

Die erfindungsgemässen neuen Verbindungen sind therapeutisch verwendbar und stellen Zwischenprodukte für die Herstellung von therapeutisch verwendbaren Verbindungen dar. Sie zeichnen sich bei relativ geringer Toxizität vor allem durch ihre lange anhaltende antiallergische, antihistaminerge und antiserotoninerge Wirkung aus, ferner hemmen sie die Blutplättchenaggregation. Therapeutische Anwendungsmöglichkeiten für die neuen Verbindungen sind z.B. die Behandlung von Reaktionen, die durch Freisetzung von Histamin oder Serotonin erzeugt wurden, Asthma bronchiale, allergische Bronchitis, allergische Rhinitis, allergische Konjunctivitis sowie allergische Diathesen. Von besonderer Bedeutung für therapeutische Zwecke ist die gute orale Wirkung der Verbindungen. Die orale Wirkung ist auch ein wesentlicher Vorteil gegenüber dem Dinatriumsalz der Cromoglicinsäure, einem vielbenutzten Handelsprodukt zur Behandlung von Asthma bronchiale und allergischer Bronchitis.

In der DE-A-2 311 019 sind Oxazepine und Diazepine beschrieben, die den erfindungsgemässen Verbindungen strukturell ähneln. Diese bekannten Verbindungen sind jedoch als antidepressiv charakterisiert, während die erfindungsgemässen sich durch antiallergische, antihistaminerge und antiserotonische Wirkung auszeichnen. Bei den Verbindungen gemäss der DE-A-2 248 477 enthält das Ringsystem, wie bei den hier beschriebenen Verbindungen, einen Fünfring mit zwei Stickstoffatomen als Ringgliedern. Die neuen Verbindungen weisen jedoch ein weiteres Stickstoffatom auf, so dass sich insgesamt eine Guanidingruppierung als Teilelement ergibt. Die Guanidinstruktur findet sich zwar in einzelnen $H_2$-Antihistaminika, jedoch auch in zahlreichen Arzneistoffen anderer Wirkungsrichtungen. In Übereinstimmung damit heisst es in «Burger's Medicinal Chemistry», 4. Auflage, Teil III, S. 509, als Resumée der Unternehmungen an zahlreichen Guanidinderivaten, «that the ability to antagonize histamine was not a property of guanidines per se».

An anderer Stelle ist diesem Werk zu entnehmen (S. 518/519), dass die Tautomeriefähigkeit für die Guanidingruppierung gegeben sein sollte, damit sich die für Antihistaminika erwünschte flache Konformation der Guanidinstruktureinheit ergebe. Bei den erfindungsgemässen Verbindungen ist die Tautomerie nicht möglich, da die Ringstickstoffatome tertiäre Aminogruppen darstellen. Dennoch findet sich überraschenderweise bei den neuen Verbindungen eine starke antihistaminerge Wirkung.

Die Verbindungen nach der DE-A-2 548 045 enthalten in ihrem Ringsystem einen Pyrrolidinring; die als Ringglied fungierende $=NR_6$-Gruppe der erfindungsgemässen Verbindungen ist durch eine Gruppe $=CH-NR_5R_6$ ersetzt. Weiterhin fehlt die doppelt an den Ring gebundene Gruppe $=NR_5$, deren Platz in den bekannten Verbindungen von einem Sauerstoffatom oder von zwei Wasserstoffatomen eingenommen wird. Die Verbindungen gemäss der vorliegenden Erfindung wurden daher durch die DE-A-2 548 045 nicht nahegelegt.

Für die Anwendung werden die erfindungsgemässen Verbindungen in üblicher Weise mit Hilfs- und Trägerstoffen zu gebräuchlichen galenischen Zubereitungen verarbeitet, z.B. zu Kapseln, Tabletten, Dragées, Lösungen, Suspensionen für die orale Anwendung; zu Aerosolen für die pulmonale Gabe; zu sterilen isotonischen wässrigen Lösungen für die parenterale Anwendung und zu Cremes, Salben, Lotionen, Emulsionen oder Sprays für die lokale Applikation.

Die Einzeldosis bei der oralen Anwendung beträgt beim Erwachsenen etwa 0,2 bis 40, vorzugsweise etwa 0,5 bis 10 mg. Für die Inhalation werden Einzeldosen zwischen 0,05 bis 20 vorzugsweise 0,2 bis 5 mg appliziert, wobei übliche Zubereitungen verwendet werden, vor allem Dosieraerosole und Kapseln für die Pulverinhalation. Die angegebenen Dosen können gegebenenfalls mehrmals pro Tag verabreicht werden.

Zur Ermittlung dieser Wirkung der erfindungsgemässen Verbindungen wurden diese zahlreichen pharmakologischen Prüfungen unterworfen.

(a) Versuche an allergisierten Ratten wurden durchgeführt nach passiver Sensibilisierung der Tiere mit IgE-Antikörpern und anschliessender Antigenprovokation. Auf diese Weise konnte eine passive cutane Anaphylaxie (GOOSE et al. (1969): Immunology 16, 749) (PCA) erzeugt werden.

(b) Antihistaminische Wirkung:

Die Verbindungen hemmten p.o. und i.v. in Ratten, Hunden und Affen die durch intrakutane Injektion von Histamin erzeugte Histaminquaddel. Die Quantifizierung erfolgte nach Extravasation von Evans Blau-Farbstoff in die Haut durch Ausmessen der Quaddel.

Als Beispiel für die Wirkung einer wichtigen erfindungsgemässen Verbindung sollen die Angaben in der nachstehenden Tabelle dienen:

| Verbindung | PCA $ED_{50}$ [mg/kg] (Ratte p.o.) | Histaminquaddel (Ratte) i.v. | p.o. | $LD_{50}$ [mg/kg] (Maus p.o.) |
|---|---|---|---|---|
| Beispiel 2 | 1,1 | 0,07 | 2,7 | 280 |

Nachstehend sind einige Beispiele für pharmazeutische Präparate mit erfindungsgemässen Wirkstoffen angegeben:

Tabletten
Zusammensetzung:

| | |
|---|---|
| Wirkstoff gemäss Formel I | 0,005 g |
| Stearinsäure | 0,001 g |
| Traubenzucker | 0,194 g |

Inhalationsaerosol
Zusammensetzung:

| | |
|---|---|
| Wirkstoff gemäss Formel I | 1,00 Teile |
| Sojalecithin | 0,20 Teile |
| Treibgasmischung (Frigen 11, 12 und 114) | ad 100,00 Teile |

Die Zubereitung wird vorzugsweise in Aerosolbehälter mit Dosierventil abgefüllt, der einzelne Hub wird so bemessen, dass eine Dosis von 0,5 mg abgegeben wird. Für die anderen Dosierungen des angegebenen Bereichs verwendet man zweckmässig Zubereitungen mit höherem oder niedrigerem Wirkstoffanteil.

Kapseln für die Inhalation

Ein Wirkstoff nach Formel I wird in mikronisierter Form (Teilchengrösse im wesentlichen zwischen 2 und 6 µm), gegebenenfalls unter Zusatz von mikronisierten Trägerstoffen, etwa Lactose, in Hartgelatinekapseln gefüllt. Zur Inhalation dienen übliche Geräte für die Pulverinhalation. In jede Kapsel werden z.B. zwischen 0,2 und 20 mg Wirkstoff und 0 bis 40 mg Lactose eingefüllt.

Salbe

| Zusammensetzung: | g/100 g Salbe |
|---|---|
| Wirkstoff gemäss der Erfindung | 2,000 |
| rauchende Salzsäure | 0,011 |
| Natriumpyrosulfit | 0,050 |
| Gemisch aus gleichen Teilen Cetylalkohol und Stearylalkohol | 20,000 |
| weisse Vaseline | 5,000 |
| künstliches Bergamotteöl | 0,075 |
| destill. Wasser | ad 100,000 |

Die Bestandteile werden in üblicher Weise zu einer Salbe verarbeitet.

Die nachstehenden Beispiele sollen die Erfindung näher erläutern. Alle Temperaturangaben sind in °C gemacht.

Herstellungsbeispiele

Beispiel 1

2-Methyl-3-imino-1,2,9,13b-tetrahydro-3-H-dibenz[c,f]-imidazo-[1,5-a]-azepinhydrobromid

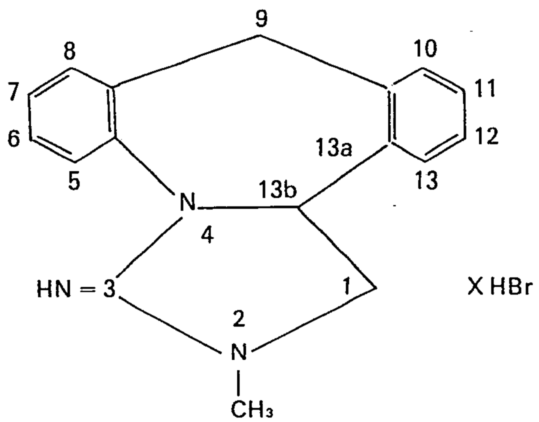

Unter Rühren wird eine Suspension von 7,15 g (0,03 Mol) 6-Methylaminomethyl-6,11-dihydro-5H-dibenz-[b, e]-azepin[Formel II; $R_1$ bis $R_4$ = H; $R_6$ = $-CH_3$; X = $-CH_2-$; gestrichelte Linie = Einfachbindung liegt vor] in 70 ml absolutem Äthanol mit einer Lösung von 3,2 g (0,03 Mol) Bromcyan in 8 ml absolutem Tetrahydrofuran versetzt. Hierbei entsteht bei leicht exothermer Reaktion eine Lösung, die nach vier Stunden bei Raumtemperatur nachgerührt wird. Anschliessend wird die Reaktionslösung mit Äther versetzt. Die anfallenden Kristalle werden abgesaugt und getrocknet. Ausbeute: 8,6 g (83% d. Th.); Fp. 247–249°C. Nach dem Umkristallisieren aus Methanol/Essigester schmilzt die analysenreine Substanz bei 247–250°C.

Beispiel 2

2-Methyl-3-imino-2,3-dihydro-9H-dibenz-[c,f]-imidazo-[1,5-a]-azepinhydrobromid

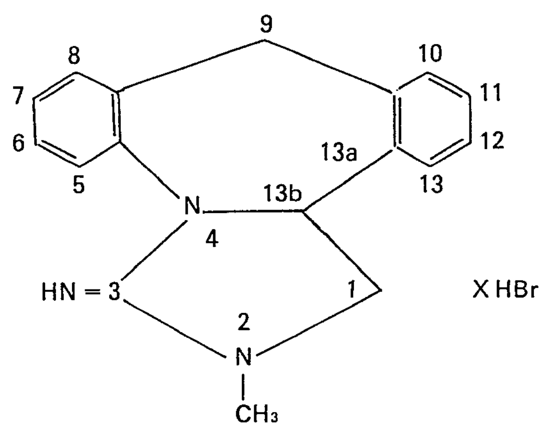

Zu einer Lösung von 44,4 g (0,188 Mol) 6-Methylaminomethyl-morphanthridin [Formel II: gestrichelte Linie = Doppelbindung; $R_1$ bis $R_4$ = H; $R_6$ = $-CH_3$; X = $CH_2$] wird unter Rühren und Eiskühlung eine Lösung von 19,93 g (0,188 Mol) Bromcyan in 140 ml absolutem Tetrahydrofuran zugetropft. Die Reaktionsmischung wird noch vier Stunden bei Raumtemperatur nachgerührt und anschliessend mit Essigester versetzt. Die anfallenden Kristalle werden abgesaugt und getrocknet.
Ausbeute: 49,5 g (77% d.Th.); Fp.: 287–289°C

Das analysenreine Hydrobromid hat nach dem Umkristallisieren aus Äthanol/Essigester einen Schmelzpunkt von 291–293°.

In analoger Weise (s. Beispiel 1 und 2) können aus den entsprechenden Diaminen der Formel II die nachstehenden Verbindungen erhalten werden:

A. Tetrahydro-Verbindungen mit X = $CH_2$, d.h. der Formel Ia

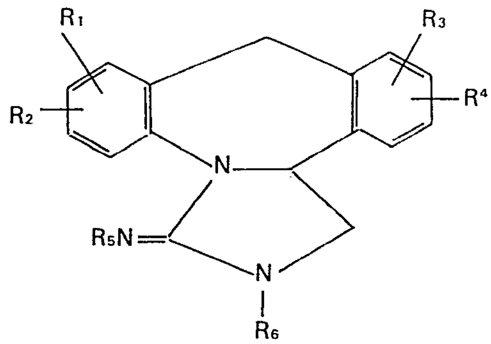

(Ia)

a) 2-Äthyl-3-imino-1,2,9,13b-tetrahydro-3H-dibenz[c,f]-imidazo-[1,5-a]-azepinhydrobromid
($R_1$ bis $R_5$ = H; $R_6$ = $-C_2H_5$)
Fp. 213–216° (Acetonitril).

b) 2-Isopropyl-3-imino-1,2,9,13b-tetrahydro-3H-dibenz-[c,f]-imidazo[1,5-a]-azepinhydrobromid
($R_1$ bis $R_5$ = H; $R_6$ =

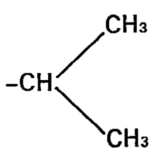

)

Fp. 230–233°
(Acetonitril/Äther).

B. Tetrahydro-Verbindungen mit X = O, d.h. der Formel Ib

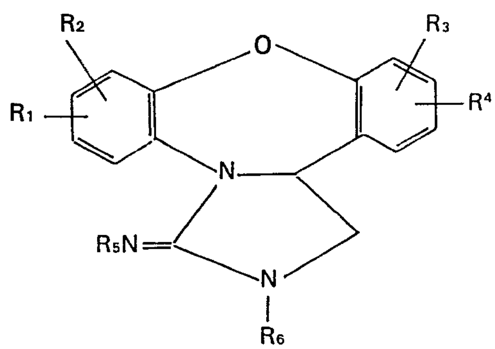

(Ib)

2-Methyl-3-imino-1,2,3,13b-tetrahydrodibenz-[b,f]-imidazo-[1,5-d][1,4]-oxazepinhydrochlorid
Fp. 297–300° (Alkohol/Essigester)
($R_1$ bis $R_5$ = H; $R_6$ = $CH_3$).

C. Tetrahydro-Verbindung mit X = S, d.h. der Formel Ic

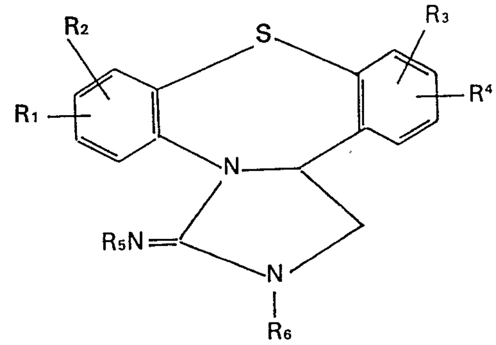

(Ic)

2-Methyl-3-imino-1,2,3,13b-tetrahydrodibenz-[b,f]-imidazo-[1,5-d][1,4]-thiazepin-hydrobromid
Fp. 204–206° (Alkohol/Essigester)
($R_1$ bis $R_5$ = H; $R_6$ = $-CH_3$).

D. Dihydro-Verbindungen mit X = $CH_2$, d.h. der Formel Id

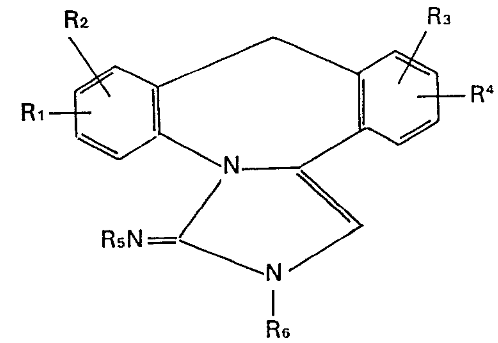

(Id)

a) 2-Äthyl-3-imino-2,3-dihydro-9H-dibenz-[c,f]-imidazo-[1,5-a]-azepin-hydrobromid ($R_1$ bis $R_5$ = H;
$R_6$ = $-C_2H_5$)
Fp. 279–282° (Methanol/Äther) (Zersetzung).

b) 2-Allyl-3-imino-2,3-dihydro-9H-dibenz-[c,f]-imidazo-[1,5-a]-azepin-hydrobromid
($R_1$ bis $R_5$ = H; $R_6$ = $-CH_2-CH=CH_2$)
Fp. 244–246° (Methanol/Äther).

c) 2-Isopropyl-3-imino-2,3-dihydro-9H-dibenz-[c,f]-imidazo-[1,5-a]-azepin-hydrobromid
($R_1$ bis $R_5$ = H; $R_6$ =

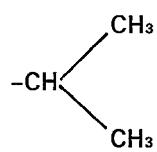

)

Fp.: 291–294°C;
(Acetonitril)(Zersetzung).

E. Dihydro-Verbindung mit X = O, d.h. der Formel Ie

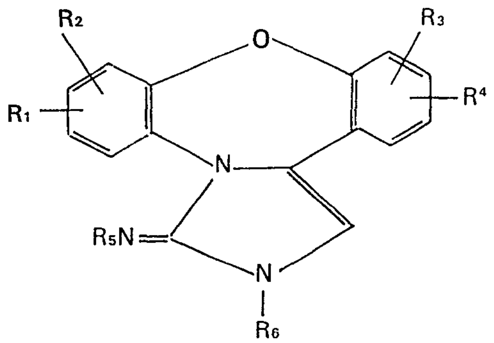

(Ie)

2-Methyl-3-imino-2,3-dihydro-dibenz-[b,f]-imidazo-[1,5-d][1,4]-oxazepin-hydrobromid
($R_1$ bis $R_5$ = H, $R_6$ = $CH_3$)
Fp. 296–299°; (Methanol/Essigester)

F. Dihydro-Verbindung mit X = S, d.h. der Formel If

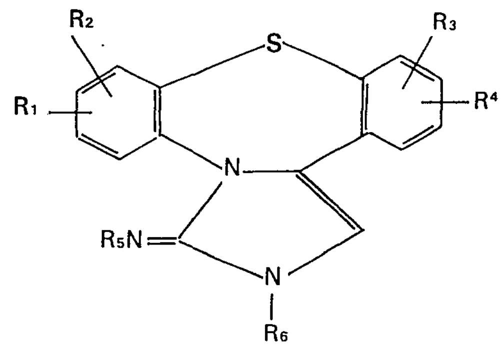

(If)

2-Methyl-3-imino-2,3-dihydro-dibenz-[b,f]-imidazo-[1,5-d][1,4]-thiazepin-hydrobromid
$R_1$ bis $R_5$ = H; $R_6$ = $CH_3$)
Fp. 314–317°C (Methanol/Essigester) Zersetzung.

Gemäss den oben beschriebenen Verfahren können auch die Verbindungen erhalten werden, die in den nachstehenden Tabellen aufgeführt sind.

Tabelle I
Verbindungen der Formel I mit einer Doppelbindung in 1/13 b-Position

| X | $R^1$ | $R^2$ | $R^3$ | $R^4$ | $R^5$ | $R^6$ | Physikal. Kenndaten |
|---|---|---|---|---|---|---|---|
| $-CH_2-$ | -H | 7-Cl | -H | -H | -H | $-CH_3$ | |
| $-CH_2-$ | $6-CH_3$ | -H | -H | -H | -H | $-CH_3$ | |
| $-CH_2-$ | 6-Cl | -H | -H | -H | -H | $-C_2H_5$ | |
| -O- | -H | -H | -H | -H | -H | $-n-C_4H_9$ | |
| -O- | $6-CH_3$ | -H | -H | -H | -H | $-CH_3$ | |
| -O- | $6-CH_3$ | -H | -H | -H | -H | $-C_2H_5$ | |
| -O- | $6-CH_3$ | -H | -H | 12-Cl | -H | $-CH_3$ | |
| -O- | 6-Cl | -H | -H | -H | -H | $-CH_3$ | |
| -O- | 6-Cl | -H | $11-CH_3$ | -H | -H | $-CH_3$ | |
| -O- | -H | 7-Cl | -H | -H | -H | 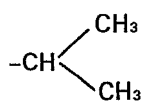 | |
| -O- | -H | -H | -H | 12-Cl | -H | $-CH_3$ | |
| -O- | -H | -H | -H | $12-CH_3$ | -H | $-CH_3$ | |
| -S- | -H | -H | -H | 12-Cl | -H | $-CH_3$ | |
| -S- | -H | -H | -H | $12-CH_3$ | -H | $-CH_3$ | |
| -S- | -H | $7-CH_3$ | -H | H | -H | $-CH_3$ | |
| -S- | $6-CH_3$ | -H | -H | -H | -H | $-CH_3$ | |
| -S- | $6-CH_3$ | -H | -H | -H | -H | $-C_2H_5$ | |
| -S- | $6-CH_3$ | -H | -H | $12-CH_3$ | -H | $-CH_3$ | |
| -S- | 6-Cl | -H | -H | -H | -H | $-CH_3$ | |

Tabelle II
Verbindungen der Formel I mit einer Einfachbindung in 1/13 b-Position

| X | $R^1$ | $R^2$ | $R^3$ | $R^4$ | $R^5$ | $R^6$ | Physikal. Kenndaten |
|---|---|---|---|---|---|---|---|
| $-CH_2-$ | -H | -H | -H | -H | $-CH_3$ | $-CH_3$ | |
| $-CH_2-$ | -H | 7-Cl | -H | -H | -H | $-CH_3$ | |
| $-CH_2-$ | $6-CH_3$ | -H | -H | -H | -H | $-CH_3$ | |

Tabelle II (Fortsetzung)
Verbindungen der Formel I mit einer Einfachbindung in 1/13 b-Position

| X | R[1] | R[2] | R[3] | R[4] | R[5] | R[6] | Physikal. Kenndaten |
|---|---|---|---|---|---|---|---|
| -O- | -H | -H | -H | -H | $-C_2H_5$ | $-CH_3$ | |
| -O- | $6-CH_3$ | -H | -H | -H | -H | $-CH_3$ | |
| -O- | $6-CH_3$ | -H | -H | -H | -H | $-C_2H_5$ | |
| -O- | $6-CH_3$ | -H | -H | 12-Cl | -H | $-CH_3$ | |
| -O- | 6-Cl | -H | -H | -H | -H | $-CH_3$ | |
| -O- | 6-Cl | -H | $11-CH_3$ | -H | -H | $-CH_3$ | |
| -O- | -H | 7-Cl | -H | -H | -H | $-CH_3$ | |
| -O- | -H | -H | -H | 12-Cl | -H | $-CH_3$ | |
| -O- | -H | -H | -H | $12-CH_3$ | -H | $-CH_3$ | |
| -S- | -H | -H | -H | 12-Cl | -H | $-CH_3$ | |
| -S- | -H | -H | -H | $12-CH_3$ | -H | $-CH_3$ | |
| -S- | -H | $7-CH_3$ | -H | -H | -H | $-CH_3$ | |
| -S- | $6-CH_3$ | -H | -H | -H | -H | $-CH_3$ | |
| -S- | $6-CH_3$ | -H | -H | -H | $-CH_3$ | $-C_2H_5$ | |
| -S- | $6-CH_3$ | -H | -H | $12-CH_3$ | -H | $-CH_3$ | |
| -S- | 6-Cl | -H | -H | -H | -H | $-CH_3$ | |

**Patentansprüche**

1. Verbindungen der allgemeinen Formel

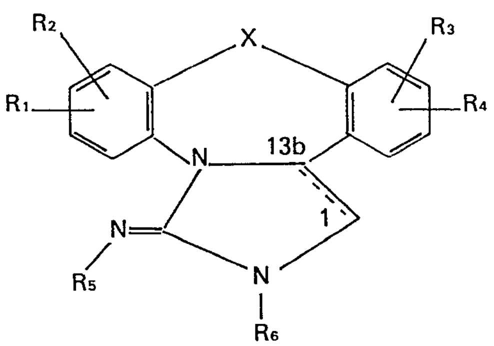

(I),

in der
$R_1$, $R_2$, $R_3$ und $R_4$ Wasserstoff, Halogen, Alkyl- oder Alkoxy mit jeweils 1 bis 6 C-Atomen,
$R_5$ Wasserstoff, Alkyl mit 1 bis 6 C-Atomen oder Allyl,
$R_6$ Alkyl mit 1 bis 6 C-Atomen, Allyl oder einen Rest der Formel

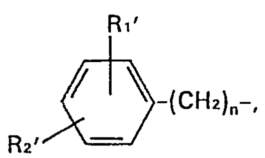

wobei $R_1'$ und $R_2'$ die gleichen Bedeutungen wie $R_1$ und $R_2$ annehmen können, aber nicht jeweils mit diesen identisch sein müssen, und n eine der ganzen Zahlen 0 bis 4 bedeutet,
X für O, S oder $CH_2$ steht und die gestrichelte Linie das Vorliegen entweder einer Einfach- oder einer Doppelbindung bedeuten kann, jeweils als freie Base oder als Säureadditionssalz.

2. Verbindungen nach Anspruch 1, in denen X die oben genannte Bedeutung hat, $R_1$ bis $R_5$ Wasserstoff und $R_6$ Alkyl mit 1 bis 3 C-Atomen bedeuten.

3. Arzneimittel, gekennzeichnet durch einen Gehalt an einer oder mehreren Verbindungen nach Anspruch 1 neben üblichen Hilfsstoffen.

4. Verwendung von Verbindungen nach Anspruch 1 als Arzneistoffe.

5. Verfahren zur Herstellung von Verbindungen der allgemeinen Formel

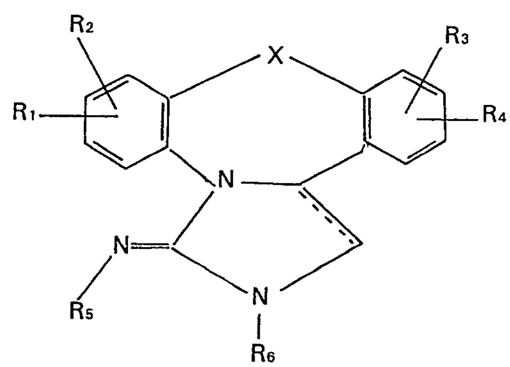

(I),

in der
$R_1$, $R_2$, $R_3$ und $R_4$ Wasserstoff, Halogen, Alkyl- oder Alkoxy mit jeweils 1 bis 6 C-Atomen,
$R_5$ Wasserstoff, Alkyl mit 1 bis 6 C-Atomen oder Allyl,
$R_6$ Alkyl mit 1 bis 6 C-Atomen, Allyl oder einen Rest der Formel

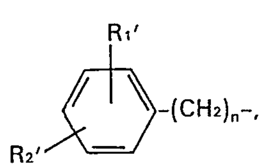

wobei $R_1'$ und $R_2'$ die gleichen Bedeutungen wie $R_1$ und $R_2$ annehmen können, aber nicht jeweils mit diesen identisch sein müssen, und n eine der ganzen Zahlen 0 bis 4 bedeutet,
X für O, S oder $CH_2$ steht und die gestrichelte Linie das Vorliegen entweder einer Einfach- oder einer Doppelbindung bedeuten kann, jeweils als freie Base oder als Säureadditionssalz, dadurch gekennzeichnet, dass man

a) zur Herstellung von solchen Verbindungen der Formel I, in denen $R_5$ Wasserstoff bedeutet, bei einem Diamin der allgemeinen Formel

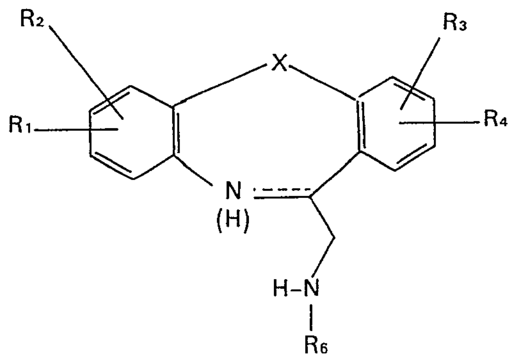

(II)

in der die gestrichelte Linie, die Reste $R_1$ bis $R_4$ und $R_6$ sowie X die obengenannte Bedeutung haben mit einem Halogencyan bzw. bei der intermediär entstehenden Verbindung der allgemeinen Formel

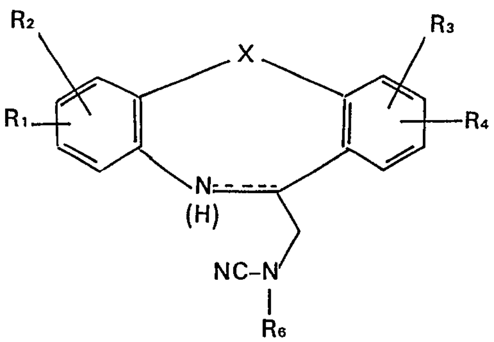

(IIa)

in der die gestrichelte Linie, die Reste $R_1$ bis $R_4$ und $R_6$ sowie X die obengenannte Bedeutung haben, durch intramolekulare Umsetzung einen Ringschluss durchführt und gewünschtenfalls zum Erhalt von solchen Verbindungen der Formel I bei denen $R_5$ Alkyl mit 1 bis 6 C-Atomen oder Alkenyl mit 3 bis 6 C-Atomen bedeutet, die wie vorstehend erhaltene Verbindung der Formel I ($R_5$ = H) mit einer Verbindung der Formel

$R_5$–X (V)

in der $R_5$ die oben genannte Bedeutung (mit Ausnahme von Wasserstoff) hat und X ein Halogenatom oder einen Sulfonyloxyrest bedeutet, in einem polaren Lösungsmittel in Gegenwart einer Base umsetzt. Oder dass man

b) bei einer Verbindung der allgemeinen Formel

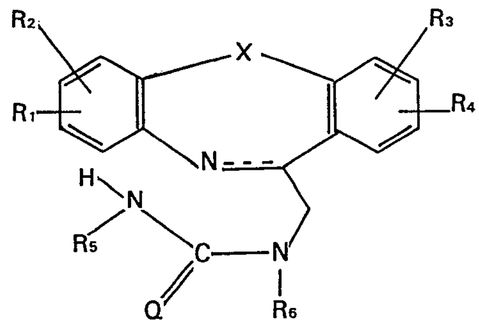

(VI)

in der die gestrichelte Linie $R_1$ bis $R_6$ sowie X die oben genannte Bedeutung haben und Q für Sauerstoff oder Schwefel steht, durch Einwirkung von Säurehalogeniden wie $POCl_3$ einen Ringschluss vornimmt, oder dass man

c) Verbindungen der allgemeinen Formel

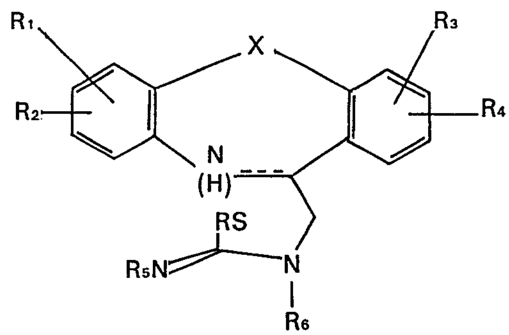

(VII)

in der die gestrichelte Linie, die Reste $R_1$ bis $R_6$ und X die oben genannte Bedeutung haben und R einen ggf. substituierten Alkylrest bedeutet, durch Erhitzen ringschliesst, und dass man gewünschtenfalls erfindungsgemäss erhaltene Racemate in die Enantiomeren aufspaltet und/oder aus primär erhaltenen freien Basen Säureadditionssalze, aus primär erhaltenen Säureadditionssalzen freie Basen herstellt.

6. Verfahren zur Herstellung von Arzneimittelzubereitungen nach Anspruch 3, dadurch gekennzeichnet, dass man einen oder mehrere Wirkstoffe der Formel I mit in der galenischen Pharmazie gebräuchlichen Hilfsstoffen zu üblichen Arzneimittelzubereitungen verarbeitet.

7. Verfahren zur Herstellung von Arzneimittelzubereitungen nach Anspruch 6, wobei die Zubereitungen Tabletten, Dragées, Kapseln, Lösungen, Suspensionen, Dosieraerosole, Sprays, Salben, Emulsionen und Cremes sind.

**Claims**

1. Compounds of general formula

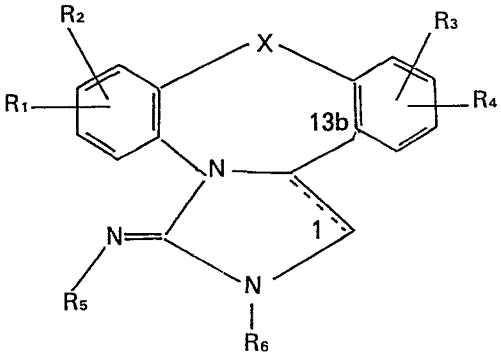

(I)

wherein

$R_1$, $R_2$, $R_3$ and $R_4$ represent hydrogen, halogen, alkyl or alkoxy each having 1 to 6 carbon atoms,

$R_5$ represents hydrogen, alkyl with 1 to 6 carbon atoms or allyl,

$R_6$ represents alkyl with 1 to 6 carbon atoms, allyl or a group of formula

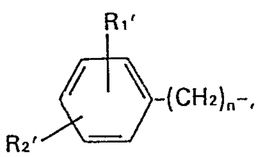

wherein $R_1'$ and $R_2'$ may have the same meanings as $R_1$ and $R_2$ but need not be identical thereto, and n represents an integer from 0 to 4,

X represents O, S or $CH_2$ and the broken line may indicate the presence of either a single or a double bond, in the form of a free base or an acid addition salt.

2. Compounds as claimed in claim 1 wherein X is as hereinbefore defined, $R_1$ to $R_5$ represent hydrogen and $R_6$ represents alkyl with 1 to 3 carbon atoms.

3. Pharmaceutical compositions, characterised in that they contain one or more compounds as claimed in claim 1 together with conventional excipients.

4. Use of compounds as claimed in claim 1 as pharmaceutical substances.

5. Process for preparing compounds of general formula

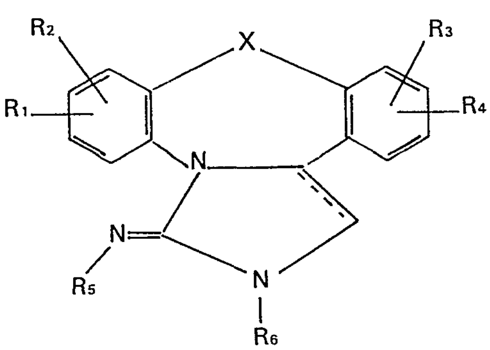

(I),

wherein

$R_1$, $R_2$, $R_3$ and $R_4$ represent hydrogen, halogen, alkyl or alkoxy each having 1 to 6 carbon atoms,

$R_5$ represents hydrogen, alkyl with 1 to 6 carbon atoms or allyl,

$R_6$ represents alkyl with 1 to 6 carbon atoms, allyl or a group of formula

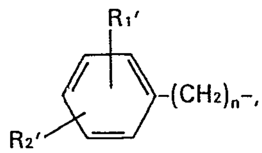

wherein $R_1'$ and $R_2'$ may have the same meanings as $R_1$ and $R_2$ but need not be identical thereto, and n represents an integer from 0 to 4,

X represents O, S or $CH_2$ and the broken line may indicate the presence of either a single or a double bond, in the form of a free base or an acid addition salt, characterised in that

a) in order to prepare compounds of formula I wherein $R_5$ represents hydrogen, cyclisation is carried out by intramolecular reaction in a diamine of general formula

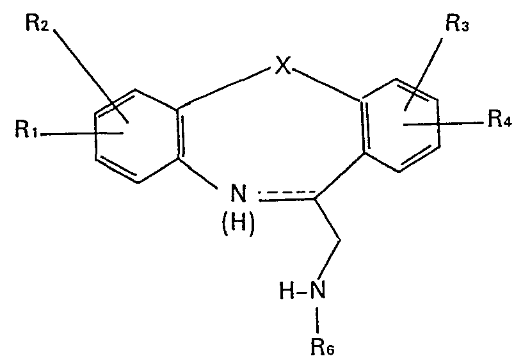

(II)

wherein the broken line, the groups $R_1$ to $R_4$ and $R_6$ and X are as hereinbefore defined, with a halocyanogen or in the compound of general formula

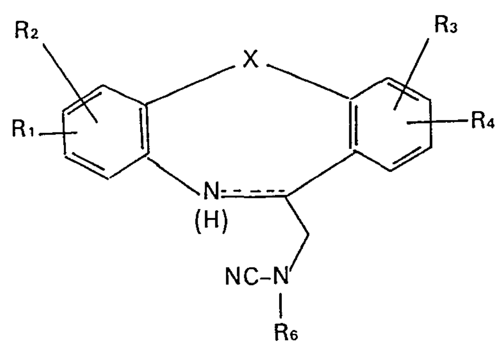

(IIa)

formed as an intermediate, wherein the broken line, the groups $R_1$ to $R_4$ and $R_6$ and X are as hereinbefore defined, and, if desired, in order to obtain compounds of formula I wherein $R_5$ represents alkyl with 1 to 6 carbon atoms or alkenyl with 3 to 6 carbon atoms, the compound of formula I ($R_5$ = H) obtained as hereinbefore is reacted with a compound of formula

$R_5$-X (V)

wherein $R_5$ has the meanings given above (with the exception of hydrogen) and X represents a halogen atom or a sulphonyloxy group, in a polar solvent in the presence of a base, or

b) in a compound of general formula

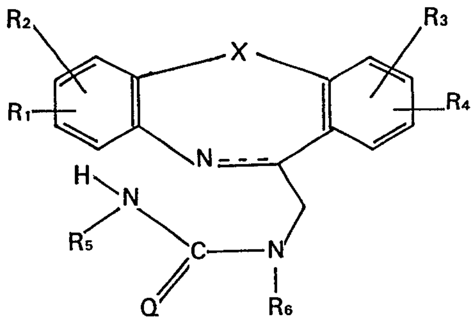

(VI)

wherein the broken line, $R_1$ to $R_6$ and X are as hereinbefore defined and Q represents oxygen or sulphur, cyclisation is carried out by the action of acid halides such as $POCl_3$, or

c) Compounds of general formula

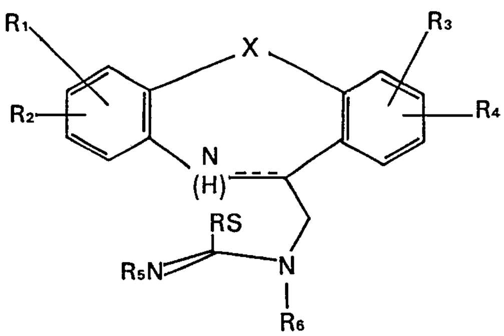

(VII)

wherein the broken line, the groups $R_1$ to $R_6$ and X are as hereinbefore defined and R represents an optionally substituted alkyl group, are cyclised by heating, and, if desired, racemates obtained according to the invention are resolved into enantiomers thereof and/or acid addition salts are prepared from free bases obtained in the first place or free bases are prepared from acid addition salts obtained in the first place.

6. Process for preparing pharmaceutical preparations as claimed in claim 3, characterised in that one or more active substances of formula I are processed with the excipients conventionally used in galenic pharmacy to obtain conventional pharmaceutical preparations.

7. Process for preparing pharmaceutical preparations as claimed in claim 6, these preparations being tablets, coated tablets, capsules, solutions, suspensions, metering aerosols, sprays, ointments, emulsions and creams.

**Revendications**

1. Composés de formule générale

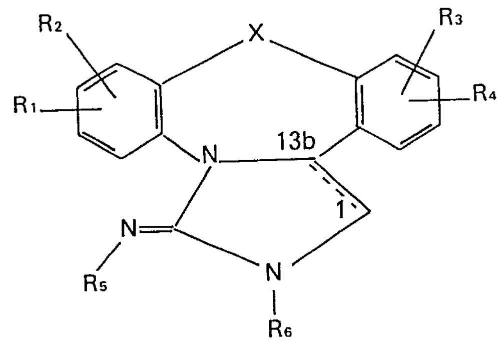

(I),

dans laquelle

$R_1$, $R_2$, $R_3$ et $R_4$ représentent hydrogène, halogène, alcoyle ou alcoxy avec chacun 1 à 6 atomes de C,

$R_5$ représente hydrogène, alcoyle avec 1 à 6 atomes de C ou allyle,

$R_6$ représente alcoyle avec 1 à 6 atomes de C, allyle ou un radical de formule

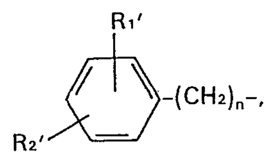

dans laquelle $R_1'$ et $R_2'$ peuvent prendre les mêmes significations que $R_1$ et $R_2$ mais ne doivent pas chacun être identiques à ceux-ci, et n représente l'un des nombres entiers 0 à 4,

X remplace O, S ou $CH_2$ et la ligne en traits interrompus peut signifier la présence soit d'une liaison simple soit d'une liaison double, chacun sous forme de base libre ou sous forme de sel d'addition d'acide.

2. Composés selon la revendication 1 dans lesquels X a la signification mentionnée plus haut, $R_1$ à $R_5$ représentent hydrogène et $R_6$ représente alcoyle avec 1 à 3 atomes de C.

3. Médicament, caractérisé par une teneur en un ou plusieurs composés selon la revendication 1, conjointement à des adjuvants usuels.

4. Utilisation de composés selon la revendication 1 en tant que substances de médicaments.

5. Procédé pour la préparation de composés de formule générale

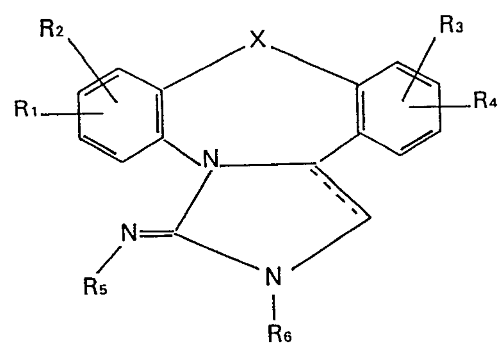

(I),

dans laquelle

$R_1$, $R_2$, $R_3$ et $R_4$ représentent hydrogène, halo-

gène, alcoyle ou alcoxy avec chacun 1 à 6 atomes de C,
$R_5$ représente hydrogène, alcoyle avec 1 à 6 atomes de C ou allyle,
$R_6$ représente alcoyle avec 1 à 6 atomes de C, allyle ou un radical de formule

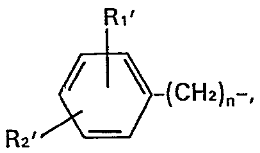

dans laquelle $R_1'$ et $R_2'$ peuvent prendre les mêmes significations que $R_1$ et $R_2$ mais ne doivent pas chacun être identiques à ceux-ci, et n représente l'un des nombres entiers 0 à 4,
X remplace O, S ou $CH_2$ et la ligne en traits interrompus peut signifier la présence soit d'une liaison simple soit d'une liaison double, chacun sous forme de base libre ou sous forme de sel d'addition d'acide, caractérisé en ce que

a) pour la préparation de composés de formule I dans lesquels $R_5$ représente hydrogène, on effectue une fermeture de cycle par réaction intramoléculaire, dans une diamine de formule générale

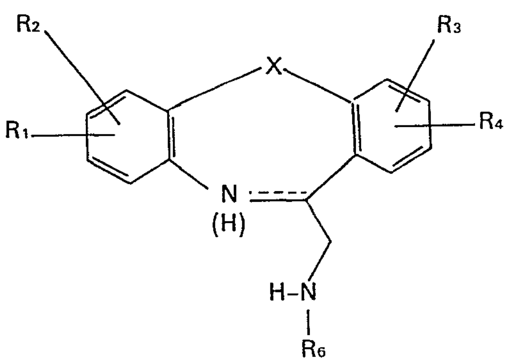

(II)

dans laquelle la ligne en traits interrompus, les radicaux $R_1$ à $R_4$ et $R_6$ ainsi que X ont la signification indiquée plus haut au moyen d'un cyanure d'halogène ou dans le composé formé de façon intermédiaire de formule générale

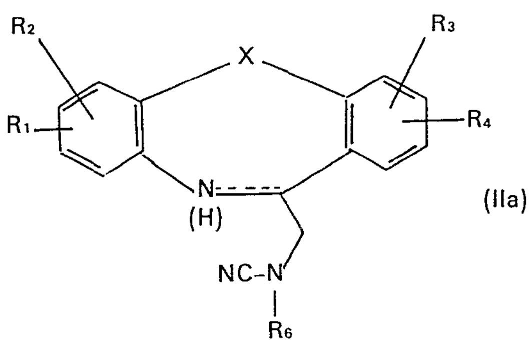

(IIa)

dans laquelle la ligne en traits interrompus, les radicaux $R_1$ à $R_4$ et $R_6$ ainsi que X ont la signification indiquée plus haut et si on le désire, pour obtenir des composés de formule I dans lesquels $R_5$ représente alcoyle avec 1 à 6 atomes de C ou alcényle avec 3 à 6 atomes de C, on fait réagir le composé de formule I ($R_5$=H) obtenu comme plus haut, avec un composé de formule

$R_5$-X (V)

dans laquelle $R_5$ a la signification mentionnée plus haut (à l'exception de l'hydrogène) et X représente un atome d'halogène ou un radical sulfonyloxy, dans un solvant polaire, en présence d'une base, ou en ce que

b) dans un composé de formule générale

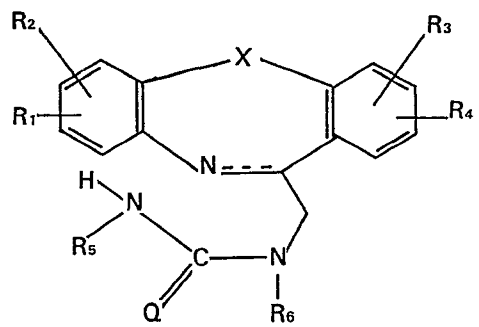

(VI)

dans laquelle la ligne en traits interrompus, $R_1$ à $R_6$ ainsi que X ont la signification mentionnée plus haut et Q remplace oxygène ou soufre, on effectue une fermeture de cycle en faisant agir des halogénures d'acides tels que $POCl_3$, ou en ce que

c) on effectue une fermeture de cycle par chauffage de composés de formule générale

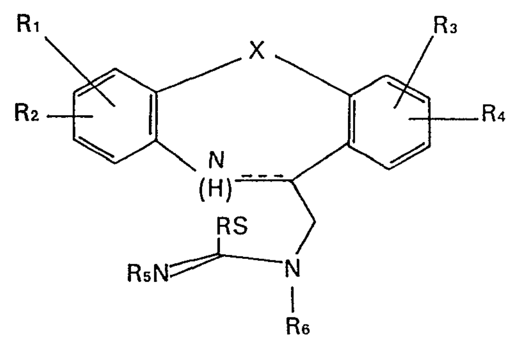

(VII)

dans laquelle la ligne en traits interrompus, les radicaux $R_1$ à $R_6$ et X ont la signification indiquée plus haut, et R représente un radical alcoyle éventuellement substitué, et en ce que, si on le désire, on clive des racémates obtenus en les énantiomères et/ou on prépare des sels d'addition d'acides à partir de bases libres obtenues en premier, des bases libres à partir de sels d'addition d'acides obtenus en premier.

6. Procédé pour la fabrication de préparations médicamenteuses selon la revendication 3, caractérisé en ce que l'on transforme une ou plusieurs substances actives de formule I en préparations médicamenteuses usuelles au moyen d'adjuvants usuels en pharmacie galénique.

7. Procédé pour la fabrication de préparations médicamenteuses selon la revendication 6, dans lequel les préparations sont des comprimés, dragées, capsules, solutions, suspensions, aérosols délivrés par des appareils doseurs, produits pour pulvérisation (sprays), onguents, émulsions et crèmes.